# EUROPEAN PATENT APPLICATION

(11) **EP 2 145 580 A1**
(43) Date of publication of application: **20.01.2010**
(21) Application number: 09164403.9
(22) Date of filing: 02.07.2009
(51) Int. Cl.: A61B 5/00, G06F 19/00, A61B 8/00

(54) **Recovery function of parameters in an ultrasound system**

(30) Priority: 14.07.2008 KR 20080068293
(71) Applicant: MEDISON CO., LTD., Gangwon-do 250-875 (KR)
(72) Inventor: Shin, Soo Hwan, 1003 Daechi-dong, Gangnam-gu 135-851, Seoul (KR); Park, Sung In, 1003 Daechi-dong, Gangnam-gu 135-851, Seoul (KR); Lee, Su Myeong, 1003 Daechi-dong, Gangnam-gu 135-851, Seoul (KR)
(74) Representative: Schmid, Wolfgang

(57) **Abstract**

Embodiments for performing a recovery function of parameters in an ultrasound system are disclosed. An input unit may allow a user to input instructions. The instructions include a setting instruction for setting values of a plurality of parameters and a recovery instruction for recovering the parameter values to previously set parameter values. An ultrasound diagnostic unit forms an ultrasound image of a target object based on the set parameter values. A storage unit may sequentially and accumulatively store the parameter values. A control unit, which is responsive to the recovery instruction, retrieves the parameter values from the storage unit and recovers the set parameter values to previous parameter values based on the retrieved parameter values. The ultrasound diagnostic unit forms an ultrasound image based on the recovered parameter values.

## Description

The present application claims priority from Korean Patent Application No. 10-2008-0068293 filed on July 14, 2008, the entire subject matter of which is incorporated herein by reference.

### TECHNICAL FIELD

The present disclosure generally relates to ultrasound systems, and more particularly to a recovery function of parameters in an ultrasound system.

### BACKGROUND

Recently, an ultrasound system has been used extensively in the medical field due to its non-invasive and non-destructive nature. Modem high-performance ultrasound imaging systems and techniques are commonly used to produce two-dimensional ultrasound images and three-dimensional ultrasound images of internal features of patients.

Generally, an ultrasound system provides a plurality of image parameters such as gain, depth, focus, zoom, scale and the like to optimize an ultrasound image. The values of the image parameters are set by a user for image optimization. The conventional ultrasound system stores only the values of the image parameters, which are finally set to optimize the ultrasound image. Thus, when a mistake occurs during the image optimization, the user must memorize the previously set image parameter values to recover the values of the image parameter to previously set values. However, since numerous image parameters are provided and adjusted for image optimization, it is difficult to memorize all of the previously set values of the image parameters.

### SUMMARY

Embodiments for a recovery function of parameters in an ultrasound system are disclosed herein. In one embodiment, by way of non-limiting example, an ultrasound system comprises: an input unit for allowing a user to input instructions, the instructions including a setting instruction for setting values of a plurality of parameters and a recovery instruction for recovering the parameter values to previously set parameter values; an ultrasound diagnostic unit operable to form an ultrasound image of a target object based on the set parameter values; a storage unit for sequentially and accumulatively storing the parameter values; and a control unit responsive to the recovery instruction and being operable to retrieve the parameter values from the storage unit and recover the set parameter values to previous parameter values based on the retrieved parameter values, wherein the ultrasound diagnostic unit is further operable to form an ultrasound image based on the recovered parameter values.

In another embodiment, a method of performing a recovery function in an ultrasound system, comprises:
a) allowing a user to input instructions, the instructions including a setting instruction for setting values of a plurality of parameters and a recovery instruction for recovering the set parameter values to previously set parameter values;
b) forming an ultrasound image of a target object based on the set parameter values;
c) sequentially and accumulatively storing the parameter values to a storage unit; and
d) in responsive to the recovery instruction, retrieving the parameter values from the storage unit and recovering the set parameter values to previous parameter values based on the retrieved parameter values.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

FIG. 1 is a block diagram showing an illustrative embodiment of an ultrasound system.
FIG.2 is a schematic diagram showing an example of a display of history information.

### DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

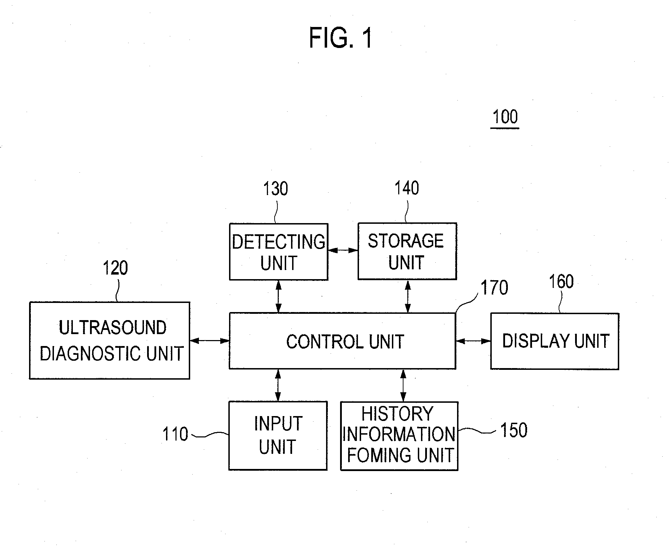

FIG 1 is a block diagram showing an illustrative embodiment of an ultrasound system 100. Referring to FIG. 1, the ultrasound system 100 may include an input unit 110 for allowing a user to input instructions. The instructions may include a selection instruction for selecting one of the diagnostic modes and a setting instruction for setting values of parameters. The diagnostic modes may include a brightness mode, a power Doppler mode, a color flow mode and the like. However, they are not limited thereto.

The parameters may include image parameters to be applied to an ultrasound image for optimization thereof. The image parameters may include gain, dynamic range, depth, focus, zoom, scale and the like, although they are not limited thereto. The parameters may further include measure parameters used to measure a size of the target object such as a distance, trace, ellipse, hip joint, etc. on the ultrasound image. However, the parameters are not limited thereto. The setting instruction may include a first setting instruction to set values of the image parameters and a second setting instruction to set values of the measure parameters. The input unit 110 may be a touch screen, a track ball, a mouse, a keyboard or the like, although it is not limited thereto.

The ultrasound system 100 may further include an ultrasound diagnostic unit 120 coupled to the input unit 110. The ultrasound diagnostic unit 120 may be operable to transmit ultrasound signals to a target object and receive echo signals reflected from the target object according to the diagnostic mode selected in response to the selection instruction inputted through the input unit 110. The ultrasound diagnostic unit 110 may be operable to form an ultrasound image of the target object based on the received echo signals. The ultrasound diagnostic unit 110 may be configured to apply values of the image parameters, which are set in response to the first setting instruction inputted through the input unit 110, to the ultrasound image for optimization thereof. The ultrasound diagnostic unit 120 may be further operable to perform a measure function using a caliper on the ultrasound image of the target object in response to the second setting instruction to thereby output measure results.

The ultrasound system 100 may further include a detecting unit 130. The detecting unit 130 may be operable to detect the input of the setting instructions and obtain the parameter values, which are set in response to the setting instructions.

The ultrasound system 100 may further include a storage unit 140. The storage unit 140 may sequentially and accumulatively store the set parameter values, which are obtained through the detection unit 130. In one embodiment, the storage unit 140 may be a data logger for sequentially recording the values of the parameters, which are set in response to the setting instructions.

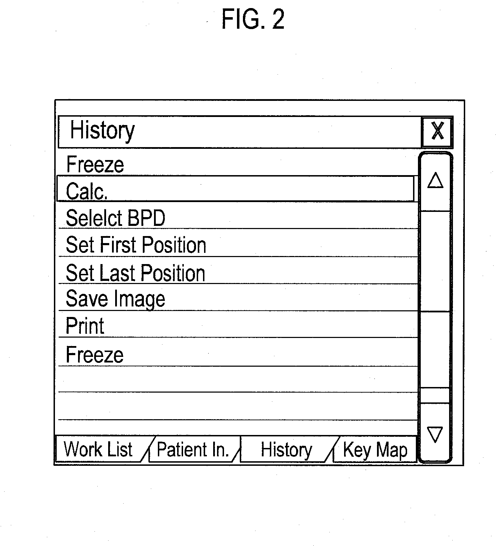

The ultrasound system 100 may also include a history information forming unit 150. The history information forming unit 150 may be operable to retrieve the values of the parameters from the storage unit 140 to thereby form history information based thereon. In one embodiment, the history information may include parameters corresponding to the stored values in the storage unit 140. The parameters included in the history information may be shown in a storing order of the parameter values, as shown in FIG. 2. The history information may be also stored in the storage unit 140.

The ultrasound system 100 may further include a display unit 160. The display unit 160 may display the ultrasound image and the measure results. The measure results may be displayed with texts, graphics or the like. The display unit 160 may also display the history information. The display unit 160 may be a liquid crystal display, a cathode ray tube display, a plasma display or an electro luminescence display. However, it is not limited thereto,

The ultrasound system 100 may additionally include a control unit 170. The control unit 170 may be operable to control the ultrasound diagnostic unit 120 to form the ultrasound image and perform the measure function in response to the inputted instructions. The control unit 170 may be configured to further operate with the history information forming unit 150 to form the history information.

In one embodiment, a request instruction for displaying the history information on the display unit 160 may further be inputted through the input unit 110. The control unit 170 may be further operable to retrieve the history information in response to the request instruction inputted from the storage unit 140. The control unit 170 may be further operable to control the display unit 160 to display the history information thereon. When the history information is displayed on the display unit 160, a recovery instruction for selecting one of the parameters on the history information may be further inputted through the input unit 110. If one of the parameters from the displayed history information is selected in response to the recovery instruction, then the control unit 170 may be operable to retrieve the previously set parameter values up to the value of the selected parameter from the storage unit 140. The ultrasound diagnostic unit 120 may be further operable to receive the retrieved parameter values and apply the received parameter values to the ultrasound image. The control unit 170 may be further operable to control the display unit 160 to display the ultrasound image with the retrieved parameter values applied.

As mentioned above, since the recovery function by using the sequentially and accumulatively stored values of the parameters is provided in one embodiment, the mistake, which may occur in a procedure for image optimization, may be reduced. Also, the user does not have to memorize the parameter values.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
an input unit for allowing a user to input instructions, the instructions including a setting instruction for setting values of a plurality of parameters and a recovery instruction for recovering the parameter values to previously set parameter values ;
an ultrasound diagnostic unit for forming an ultrasound image of a target object based on the set parameter values;
a storage unit for sequentially and accumulatively storing the parameter values; and
a control unit responsive to the recovery instruction and being configured to retrieve the parameter values from the storage unit and recover the set parameter values to previous parameter values based on the retrieved parameter values, wherein the ultrasound diagnostic unit is further configured to form an ultrasound image based on the recovered parameter values.

2. The ultrasound system of Claim 1, further comprising:
a history information forming unit for forming history information representing parameters corresponding to the parameter values stored in the storage unit in a storing order of the parameter values; and
a display unit for displaying the ultrasound image and the history information.

3. The ultrasound system of Claim 2, wherein the instructions further include a request instruction for displaying the history information on the display unit, and wherein the recovery instruction is for selecting one of the parameters on the history information.

4. The ultrasound system of Claim 3, wherein the parameters include image parameters to optimize the ultrasound image and measurement parameters to measure a size of the target object.

5. The ultrasound system of Claim 3, wherein the setting instruction includes a first instruction for setting values of the image parameters and a second setting instruction for setting values of the measurement parameters.

6. A method of performing a recovery function in an ultrasound system, comprising:
a) allowing a user to input instructions, the instructions including a setting instruction for setting values of a plurality of parameters and a recovery instruction for recovering the set parameter values to previously set parameter values;
b) forming an ultrasound image of a target object based on the set parameter values;
c) sequentially and accumulatively storing the parameter values to a storage unit; and
d) in response to the recovery instruction, retrieving the parameter values from the storage unit and recovering the set parameter values to previous parameter values based on the retrieved parameter values.

7. The method of Claim 6, further comprising:
forming history information representing parameters corresponding to the parameter values stored in the storage unit in a storing order of the parameter values; and
displaying the ultrasound image and the history information.

8. The method of Claim 7, wherein the instructions further include a request instruction for displaying the history information on the display unit, and wherein the recovery instruction is for selecting one of the parameters on the history information.

9. The method of Claim 8, wherein the parameters include image parameters to optimize the ultrasound image and measurement parameters to measure a size of the target object.

10. The method of Claim 8, wherein the setting instruction includes a first instruction for setting values of the image parameters and a second setting instruction for setting values of the measurement parameters.
